# EUROPEAN PATENT SPECIFICATION

(11) **EP 1 185 299 B1**
(45) Date of publication and mention of the grant of the patent: **17.01.2007**
(21) Application number: 00940332.0
(22) Date of filing: 07.06.2000
(51) Int. Cl.: A61K 39/395, A61K 35/28, C12N 5/10, C12N 15/85, A61P 43/00

(54) **USE OF ANTIBODIES AGAINST CD20 FOR THE TREATMENT OF THE GRAFT VERSUS HOST DISEASE**
VERWENDUNG VON GEGEN CD20 GERICHTETEN ANTIKÖRPERN ZUR BEHANDLUNG DER GRAFT VERSUS HOST KRANKHEIT
UTILISATION D'ANTICORPS CONTRE CD20 POUR TRAITER LA MALADIE DE REJET DU GREFFON

(30) Priority: 11.06.1999 IT MI991299
(43) Date of publication of application: 13.03.2002
(73) Proprietor: CONSIGLIO NAZIONALE DELLE RICERCHE, 00185 Roma (IT)
(72) Inventor: GOLAY, Josee Lab. di Ummunoematologia Molecolare, I-20157 Milano (IT); INTRONA, Martino Lab. di Immunoematologia Molec., I-20157 Milano (IT); RAMBALDI, Alessandro Divisione di Ematologia, I-24128 Bergamo (IT); BIONDI, Andrea Ctr.Ric.Tettamanti, I-20052 Monza (IT)
(74) Representative: Minoja, Fabrizio
(86) International application number: PCT/EP2000/005212
(87) International publication number: WO 2000/076542

(56) References cited:
- WO-A-97/45142
- WO-A-98/42824
- VERZELETTI SIMONA ET AL: "Herpes simplex virus thymidine kinase gene transfer for controlled graft-versus-host disease and graft-versus-leukemia: Clinical follow-up and improved new vectors." HUMAN GENE THERAPY, vol. 9, no. 15, 10 October 1998 (1998-10-10), pages 2243-2251, XP000946824 ISSN: 1043-0342
- DATABASE BIOSIS [Online] BIOSCIENCES INFORMATION SERVICE, PHILADELPHIA, PA, US; August 1998 (1998-08) MCLAUGHLIN PETER ET AL: "Rituximab chimeric anti-CD20 monoclonal antibody therapy for relapsed indolent lymphoma: Half of patients respond to a four-dose treatment program." Database accession no. PREV199800407092 XP002149247 & JOURNAL OF CLINICAL ONCOLOGY, vol. 16, no. 8, August 1998 (1998-08), pages 2825-2833, ISSN: 0732-183X
- INTRONA MARTINO ET AL: "Genetic modification of human T cells with CD20: A strategy to purify and lyse transduced cells with anti-CD20 antibodies." HUMAN GENE THERAPY, vol. 11, no. 4, 1 March 2000 (2000-03-01), pages 611-620, XP000946823 ISSN: 1043-0342

## Description

### SUMMARY OF THE INVENTION

The present invention refers to the use of antibodies against exogenous surface antigens not present on normal human T lymphocytes for the preparation of compositions for the treatment of the graft versus host disease in patients who have received T lymphocytes transduced with such exogenous surface antigens.

The invention further relates to vectors for the transfection of human T lymphocytes with exogenous surface antigens and human T lymphocytes transduced with exogenous surface antigens..

### BACKGROUND

The problem of the clinical relapses in patients with hematologic neoplasias (leukaemia and lymphomas) represents an increasingly important problem. A precise therapeutic role has been assigned for many years to the transplantation procedures with total bone marrow or with circulating purified precursors (J.O.Armitage, Bone marrow transplantation, New England Journal of Medicine, 1994, 330, 827-838). The clinical efficacy of such procedures is partially based upon a mechanism of immune recognition of the leukaemic cells of the host by the donor's T lymphocytes (GVL = Graft Versus Leukaemia) (M.Sykes, FASEB J., 10, 721-730, 1996). Nonetheless the transplants are characterised by many toxic effects including the immunologic reactivity of the donor's lymphocytes themselves against the normal tissues of the host (GVDH= Graft Versus Host Disease). In other words, the administration of T lymphocytes to the host shows clear benefits associated with severe risks and it is impossible to pharmacologically separate these two aspects.

Although standardised immunoselection techniques allow today the easy production of large quantities of purified donor's T lymphocytes for administration in order to induce in vivo the GVL effect, appropriate techniques to pharmacologically induce the selective death of the administered T lymphocytes in a patient in order to eliminate the GVHD effect in the moment in which this is clinically needed are not yet available.

In the last years many polyclonal and monoclonal antibodies have been produced against human surface molecules; in many cases antibodies have been produced with the direct aim of killing in vivo a cell positive for that molecule so to be utilised in immunotherapy protocols; as an example, limiting to the B lymphoma area, efficacious antibodies have been produced and characterised against the CD20, CD 19, CD40, CD22, CD52, CD38 molecules and yet others (P.S.Multani et al., J.Clin.Oncol., 16, 3691-3710, 1998). In some cases the antibodies directed against such molecules have shown in vivo cytotoxicity probably as they are able to activate the complement system on the surface of the target cell, as is the case with CD20, CD38, and CD52. In other cases antibodies have been conjugated with radioactive molecules to induce the target radiolysis, as is the case with CD20, Lym-1 and others. Other antibodies have been conjugated to toxins of bacterial or vegetable origin with the same aim, as is the case with CD19, CD40 and CD22. Other antibodies have been chimerised to allow a bispecificity so to bring two cells in close proximity, for example. Finally, for many of these antibodies engineered and/or humanised versions exist which allow to administer them in vivo reducing the risk of antigenicity and increasing their efficacy.

### DISCLOSURE OF THE INVENTION

It has been now found that it is possible to effectively control the graft versus host disease problem by use of a method comprising the introduction of an exogenous surface antigen in the donor's T lymphocytes and the subsequent administration to the receiving patient of the antibodies directed against such exogenous antigen.

By exogenous antigen any surface antigen not present on normal T lymphocytes is meant, as is the case with the antigens expressed on the surface of B lymphocytes such as CD20, CD 19, CD40, CD22, CD52 etc. etc. Obviously, the surface antigen will be selected so as not not cause, following the reaction with the corresponding antibody, negative or unwanted effects at the level of the cellular populations which express constitutively the antigen.

It is particularly preferred the CD20 surface antigen of the human B lymphocytes against for which a humanised monoclonal antibody is commercially available (Rituximab ®, Roche) which is used in the treatment of B non Hodgkin lymphomas.

According to the invention, donor T lymphocytes are transduced by suitable techniques with the selected antigen and are then enriched through immunoaffinity methods before being injected to the receiving subject. In case the graft versus host disease develops, the antibody against the antigen is administered in order to inactivate in vivo the T lymphocytes by use, for example, of complement mediated cytotoxic mechanisms.

The antibody will preferably be monoclonal, more preferably it will be a humanised monoclonal antibody. Dosages and administration route will depend on many factors including overall health status, weight, sex and age of the patient. Generally the antibody will be administered by iv route in a dosage range from approximately 50 to approximately 500 mg/m² of body surface, one to three times a day until the almost complete disappearance of the circulating T lymphocytes.

The isolation of T lymphocytes has been described by Rambaldi et al., Blood, 91, 2189-2196, 1998.

The methods to transduce the T lymphocytes with the desired antigen are well known: as a reference see the review by Verma I.M. and Somia N. in Nature, 389, 239-242, 1997. In particular, suitable vectors can be used, such as retroviruses, adenoviruses, adeno associated viruses, herpesviruses, lentiviruses etc. etc.

Each of these vectors includes, in its turn, many different types of organisms: considering retroviruses, examples are amphotropic, ecotropic and xenotropic vectors. Furthermore many different packaging cell lines have been utilised in the years to optimise the production of such recombinant retroviruses and to guarantee better handling and safety for the producers (I.M.Verma et al., Nature, 389, 239-242, 1997; M.A.Kay et al., Gene therapy, Proc. Natl. Acad. Sci.USA, 94, 12744-12746,1997).

Recently, also naked DNA has been introduced into target cells through conjugation with polycationic or liposomal complexes, electroporation, precipitation in salt buffers and other techniques.

Many different cell types have been targeted with genetic transfer: T and B lymphocytes, immature haematopoletic precursors, muscle cells, fibroblasts, hepatocytes and other cell types (I.M. Verma et al., Nature, 389, 239-242, 1997;M.A. Kay et al., Gene therapy, Proc. Natl. Acad. Sci. USA, 94, 12744-12746, 1997)

In the case of CD20 antigen, an amphotropic retrovirus has been used which derives from the Moloney murine leukaemia virus and is packaged in embryonic kidney human cells (293 T) engineerized to contain the retroviral structural elements on separate plasmids (Human Gene Therapy, 7, 1405-1413,1996). Such vectors, as well as the T lymphocytes transduced with the exogenous antigen, in particular the CD20+ T lymphocytes, are an object of the present invention.

After the genetic transfer the cells which express significantly the exogenous gene constitute only a minority of the total population. Selection procedures of the transduced cells are carried out, by use of exogenous genes which are able to give a selective advantage to the cell. The transduced cells can also be selected according to alternative methods such as FACS sorting with antibodies against the exogenous antigens (K.Phillips, et al., Nature Medicine, 2, 10, 1154-1155, 1996). Other methods are immunoaffinity columns or preadsorbed culture plates for the panning procedure, and the like.

### Description of the figures

Figure 1: scheme of the plasmid LTR CD20 LTR;
   LTR = long terminal repeat; pUC = plasmid origin of replication; Puro = gene which confers puromycin resistance; PGK1 = promoter of the phosphoglyceraldehyde kinase; EBNA1 and OriP = elements derived from the EBV virus for the episomal replication; AmpR = gene for the ampicillin resistance.
Figure 2: infection of the CEM cell line with CD20 and immunoselection.
   Left panel A: CEM cell line after virus infection, analysed at the cytofluorimeter with a fluorescent control IgG1 antibody.
   Central panel B: the same population analysed with a fluorescent anti CD20 antibody.
   Right panel C: the same population after immunoselection on affinity columns, analysed with a fluorescent anti CD20 antibody.

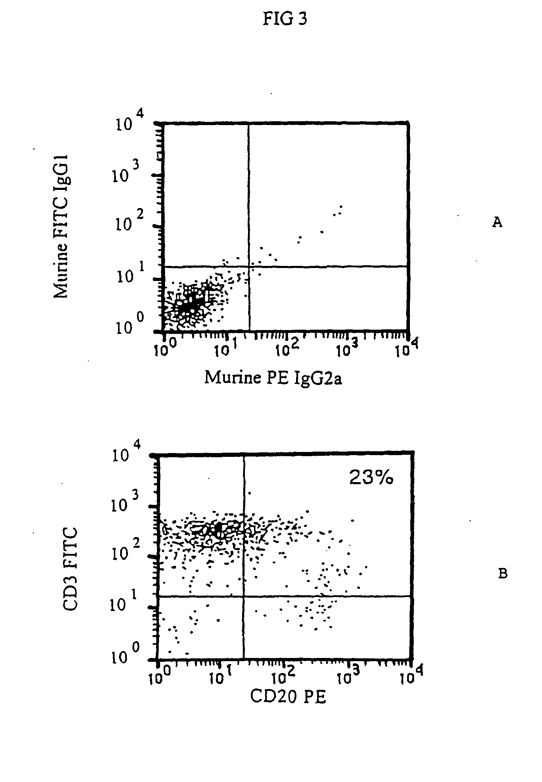

Figure 3: infection of human fresh T lymphocytes with CD20 virus
   Left panel A: after the infection the lymphocytes are labelled with PE IgG2a and FITC IgG1 control antibodies.
   Right panel B: same population is labelled with anti CD20 PE and anti CD3 FITC antibodies. In the shown case 23% of the cells are double positive.

The following examples illustrate the invention in greater detail

### Example 1

### Construction of the plasmid LTR CD20 LTR

A 913 nt fragment from the human CD20 cDNA containing the entire coding sequence has been obtained by PCR from the plasmid pCMV CD20 (Becker et al., Science, 249, 912-915, 1990).

For the amplification, 40 ng of plasmid were brought in a final reaction volume of 100 µl in 10 mM KCl, 10 mM (NH4)2SO4, 20 mM Tris HCl, pH 8.75, 2 mM MgSO₄, 0.1% Triton X-100, 100 µg/ml BSA, in the presence of 0.8 µl of a solution of 2.5 mM dNTP, 500 ng of primer "sense" (CGGGATCCAAAATGACAACACCCAGAAATTC), 500 ng of primer "antisense" (CGGGATCCTTAAGGAGAGCTGTCATTTTCT) and 5U Pfu DNA Polymerase from Stratagene (La Jolla, CA, USA). The reaction was carried out for 26 cycles in the cycler following this scheme: 1' at 95° C, 1' at 60°C and 2' at 72°C. At the end of the reaction 100 µl of a 25:24:1 phenol chloroform and isoamyl alcohol solution were added and after extraction, DNA was precipitated overnight at 20°C in the presence of ethanol. After centrifugation, DNA was resuspended in 100 µl water and then subcloned in the pMOS vector (Amersham Italia, srl, Italy) according to the manufacturer's instructions contained in the kit "pMOS blunt ended cloning kit". The resulting recombinant plasmid was amplified and sequenced, then digested with BamHI whose recognition site (G/GATCC) was present in both PCR primers' ends. Therefore the fragment was subcloned in the BamHI site of the retroviral vector PINCO VUOTO. The retroviral vector PINCO VUOTO had been previously obtained following excision with EcoRI and NotI of a 1441 bp fragment containing the CMV promoter (Cytomegalovirus) and the EGFP (enhanced green fluorescent protein) gene from the plasmid PINCO (F.Grignani e al., Cancer Res., 58, 14-19, 1998). After excision of the EcoRI-NotI fragment, the plasmid was closed after end blunting with Klenow fragment and called PINCO VUOTO. Such retroviral vector is now of 11448 bp in length.

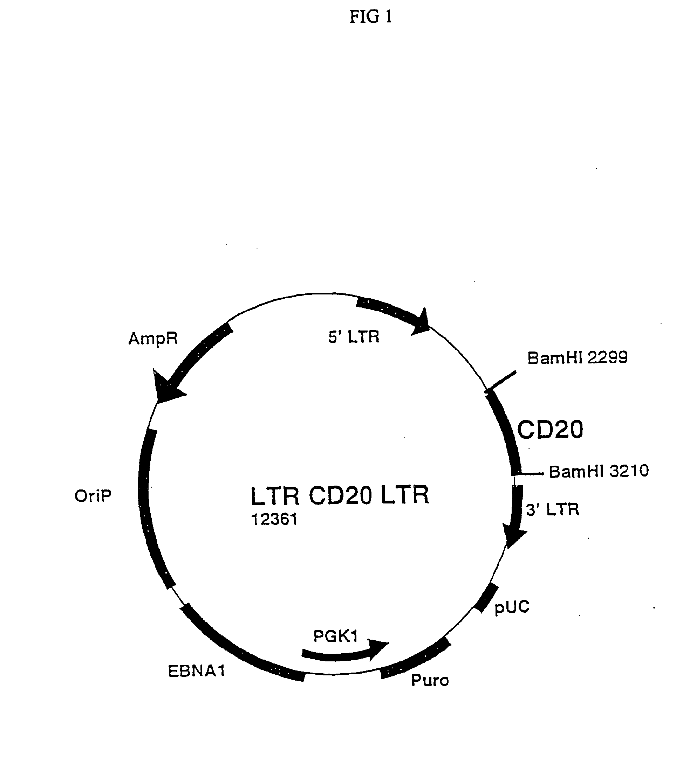

The recombinant between PINCO VUOTO and the CD20 cDNA was called LTR-CD20-LTR and sequenced to check the cloning and the integrity of the CD20 cDNA as well as the absence of stop codons upstream the first ATG (Fig.1).

The construct LTR-CD20-LTR is therefore made of, for the retroviral portion, the LTR derived from the Moloney murine leukaemia virus (MoMLV), other retroviral sequences derived from the Moloney virus, the CD20 cDNA in the BamHI site and the second LTR as detailed in annexed Fig.1. The rest of the plasmid is identical to the PINCO plasmid (F.Grignani et al., Cancer Res., 58, 14-19, 1998) which contains, as shown in the figure, EBNA-1 and OriP elements from the Epstein Barr virus, the origin of replication (pUC) and the gene for the ampicillin resistance, as well as a gene for the puromycin resistance under the control of PGK-1 promoter.

### Example 2

### Transfection of the LTR-CD20-LTR plasmid in the packaging cells

In order to produce retroviruses, the packaging cell Phoenix-Ampho was transfected with the LTR-CD20-LTR plasmid.

The Phoenix-Ampho cells are derived from the human embryonic kidney 293 cell line following several modifications; initially they were transfected with the E1A gene from adenovirus and then transfected with two separate plasmids coding for the structural genes gag and pol from Moloney MLV under the control of Rous sarcoma virus promoter and the env gene from Moloney MLV under the control of cytomegalovirus promoter.

1.5 x 10⁶ cells were plated on day -1 in a Petri dish of 10 cm diameter in 10 ml DMEM medium (Gibco, Seromed, Berlin, Germany) added with 10% FCS (Hiclone Laboratories, Steril System, Logan, UK) and kept in 5% CO₂ incubator at 37°C. On day 0 16 µl chloroquine were added (stock solution 25 mM in PBS) and after 10' 1 ml solution of 10 µg plasmid DNA was added. To obtain such DNA solution, 500 µl of a solution 2X HBS (50 mM HEPES, pH 7.05, 10 mM KCl, 12 mM Dextrose, 280 mM NaCl, 1.5 mM Na₂HPO₄ (FW 141.96)) were added in a 15 ml conic tube. Subsequently, in a second 15 ml tube, 500 µl of a solution with 10 µg DNA, 61 µl CaCl 2M and sterile water were prepared. After that, the DNA mixture was added dropwise in the first tube and the obtained precipitated was then added to the cells.

After 8 hours the medium was replaced with 10 ml of fresh DMEM.

On day +1 the medium was replaced with 5 ml fresh RPMI 1640 medium added with 10% FCS.

On day +2 the infection was carried out by removing the 5 ml of medium containing the retroviruses released during the culture.

### Example 3

### Infection of the CEM cell line with the LTR-CD20-LTR retrovirus

1 x 10⁶ human T lymphoblastoid CEM cells growing in suspension in RPMI 1640 medium supplemented with 10% FCS and glutamine, were pelleted by spinning at 1200 rpm for 8' in a flat bottom well of a 24 wells plate (Falcon, Becton Dickinson and Company, NY). After removal of the supernatant, 1 ml of the viral supernatant was added by filtration through 0.45 µm filters (Millipore Corporation Bedford, MA) in the presence of 1 µl Polybrene (stock solution 4 mg/ml in PBS).

The plate was then centrifuged for 45' at 1800 rpm at room temperature and then the supernatant was removed and replaced with 1 ml fresh RPMI 1640 added with 10% FCS and subsequently incubated for additional 6 hours.

At the end of the incubation the infection procedure was repeated a second time using a different Petri dish of packaging cells previously prepared.

### Example 4

### FACS analysis of CD20+ CEM

CEM cells following retroviral infection with LTR-CD20-LTR were kept in the incubator and normally grown in RPMI 1640 medium added with 10% FCS. After 2 days the CEM cells could already be assayed by immunofluorescence analysis for the presence of the CD20 marker on the surface.

0.1 x 10⁶ cells were transferred in an 1.5 ml Eppendorf tube, spun at 4,000 rpm for 3', resuspended in 50 µl of a solution of fluorescent anti CD20 1F5 antibody (Becton Dickinson) and kept for 30' at 4°C. At the end, 500 µl of a solution 0.9% NaCl, 5% FCS, 0.02% Na Azide were added and cells were spun at 4,000 rpm for 5'. After that, the sample was resuspended in 100 µl of PBS solution containing 1% formaldehyde and then kept at 4°C until reading at the fluorocytometer.

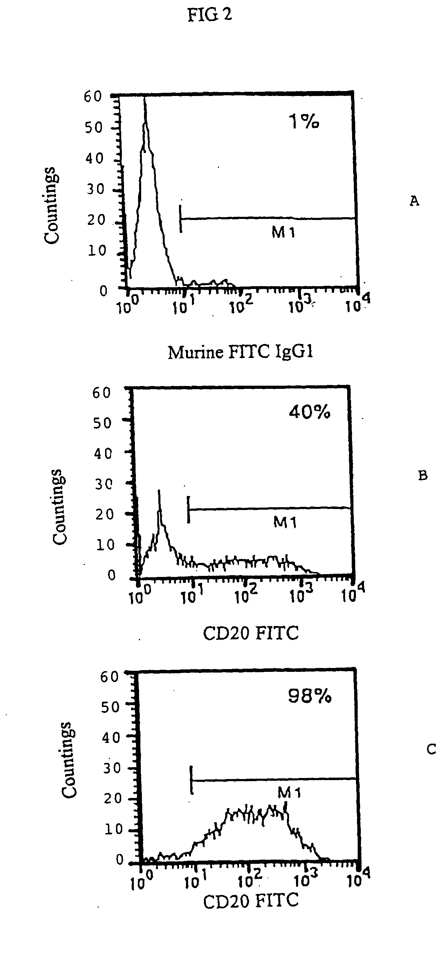

In many experiments this infection procedure always gave CEM CD20+ cells in varying percentages from 30 to 60%, while the non infected cell line was completely negative for the CD20 expression (as an example see Fig. 2, central panel, showing a CEM population which became by 40% CD20+.).

### Example 5

### Immunoaffinity separation

CEM cells infected with LTR-CD20-LTR virus after two days of culture cuold be enriched in the CD20+ population by immunoaffinity columns. To this purpose cells were first incubated for 30' at 4°C with the anti CD20 antibody clone 1F54, then washed three times with PBS and 2.5% human serum albumin, finally incubated for additional 30' at 4°C with a solution of microbeads coated with a goat anti mouse IgG antibody (Milteny Biotech, Bergish-Gladbach, Germany).

Finally the cells were resuspended in medium RPMI 1640 and selected through passage on XS+ column in the SuperMACS system (Milteny Biotech). Then the column was eluted with physiologic solution added with 2.5% albumin and the column was removed from the SuperMACS and washed in order to recover the positive fraction.

The positive fraction was further analysed at the cytofluorimeter following cell labelling according to the direct immunofluorescence procedure previously described.

The percentage of CD20+ cells at the end of this procedure has always been above 90%. As an example see Fig.2, right panel, in which a CEM population is shown after enrichment by immunoaffinity which is CD20+ positive at 98%.

At the end the CEM CD20+ population was grown in suspension and expanded in medium RPMI 1640 added with 10% FCS in incubator. At regular intervals this population was studied for the expression of the CD20 marker on the surface thus showing the stability of the marker for more than two months and the positivity on more than 90% of the selected cells.

### Example 6

### Infection with the LTR-CD20-LTR virus of peripheral fresh T lymphocytes

Heparinised total blood was stratified over Ficoll and centrifuged for 30' at 1,500 rpm at room temperature. The cells collected at the interface were washed with PBS and spun at 1,500 rpm for 10' at room temperature, then two further times at 1,000 rpm for 10' at room temperature and finally resuspended in RPMI 1640 with 10% FCS at 1 x 10⁶ / ml in 24 wells plates with flat bottoms, aliquoting 2 ml of cell suspension per well in the presence of PHA (Murex) at 1 µg/ml at 37°C and 5% CO₂ for one night.

The second day human recombinant IL-2 was added (Proleukin, Chiron Italia, Milan, Italy) at the final concentration of 100U/ml.

At the third day, after washing and cell countings, 1 x 10⁶ cells were infected in 1 ml of medium in one flat bottom well in a 24 wells plate. After spinning at 1,200 rpm for 10', the supernatant was removed and replaced with 1 ml filtered virus in the presence of polybrene and subsequent spinning for 45' at 1,800 rpm at room temperature as from the above referred protocol.

At the end, the viral supernatant was removed and replaced with complete medium for 6 hours incubation and then the spin infection procedure was repeated. After that, cells were resuspended in complete medium in the presence of Il-2 and left to stand in the incubator overnight.

The entire procedure was repeated for the following two days and finally the cells were kept in culture for two additional days in incubator.

Then the cells were labelled with monoclonal antibodies anti CD20 FITC, anti CD3 PE, anti CD4 PE, and anti CD8 FITC (Becton Dickinson) with the same procedure described above and then analysed at the cytofluorimeter.

Many experiments on normal donors show that a varying percentage from 5% to 25% of CD3+ T lymphocytes acquires the CD20 marker in double fluorescence analysis. One such experiment is shown in Fig. 3, in this specific case 23% CD3/CD20 double positivity having been attained.

### Example 7

Study of the lysis induced by antibody and complement in populations of fresh human T lymphocytes after CD20 gene transduction.

2 x 105 transduced lymphocytes were aliquoted in 10 ml round bottomed tubes in 500 µl of RPMI 1640 medium added with 10% heat inactivated foetal calf serum. Then the Rituximab antibody was added to the final concentration of 350 g/ml and rabbit Pel freeze complement at final 10%.

Alternatively, human AB serum at the final 30% concentration can be added as a source of complement. Cells were left for one hour at 37°C in a thermostatized water bath with continuous shaking. The cell suspension was added with an equal volume of 1X solution of acridine orange in PBS (stock 100 X solution consisting of 30 mg in 100 ml distilled water) and the cell suspension was evaluated at the cytofluorimeter: the living cells emit green fluorescence and were counted as percentage on the total population analysed. With this quick method, the killing efficiency of Rituximab® on the CD20+ cells could be assessed, comparing the percentages of double positive CD3/CD20 cells in the different studied populations and the percentages of dead cells after Rituximab® addition. As shown in the Table, the control populations were the same cells exposed to the antibody alone or to complement alone. Data shown in the table prove that one hour exposure to Rituximab® induces almost 90% death of the CD3/CD20 + cells.

**Table: Complement-dependent cytotoxicity of CD20 transduced fresh human T lymphocytes**

| % specific lysis | | | | |
|---|---|---|---|---|
| | % CD3/CD20+ lymphocytes | Rituximab® Alone | Complement alone | Rituximab® Plus Complement |
| Donor 1 | 30 | 0 | 14 | 33 |
| Donor 2 | 23 | 0 | 11 | 35 |
| Donor 3 | 15 | 0 | 5 | 18 |

The lysis percentage was determined at the FACS following staining with acridine.

## Claims

1. The use of antibodies against antigens expressed on the surface of B-lymphocytes and not present on human normal T lymphocytes, for the preparation of compositions for the treatment of the graft versus host disease in patients who have received T lymphocytes transduced with such antigens.

2. The use according to claim 1, wherein antibodies against the CD20 surface antigen and lymphocytes transduced with the CD20 antigen are used.

3. The use according to claim 2, wherein the anti CD20 antibody is a humanised monoclonal antibody.

4. Human T lymphocytes transduced with antigens expressed on the surface of B-lymphocytes and not present on human normal T lymphocytes

5. T lymphocytes according to claim 4 transduced with human CD20 antigen.

## Patentansprüche

1. Verwendung von Antikörpern gegen Antigene, die auf der Oberfläche von B-Lymphocyten exprimiert werden und nicht auf menschlichen normalen T-Lymphocyten vorhanden sind, für die Herstellung von Zusammensetzungen zur Behandlung der Graft-versus-Host-Krankheit bei Patienten, die T-Lymphocyten erhalten haben, die mit solchen Antigenen transduziert sind.

2. Verwendung nach Anspruch 1, wobei Antikörper gegen das CD20-Oberflächenantigen und Lymphocyten, die mit dem CD20-Antigen transduziert sind, verwendet werden.

3. Verwendung nach Anspruch 2, wobei der anti-CD20-Antikörper ein humanisierter monoklonaler Antikörper ist.

4. Menschliche T-Lymphocyten, die mit Antigenen transduziert sind, die auf der Oberfläche von B-Lymphocyten exprimiert werden und nicht auf menschlichen normalen T-Lymphocyten vorhanden sind.

5. T-Lymphocyten nach Anspruch 4, die mit menschlichem CD20-Aritigen transduziert sind.

## Revendications

1. Utilisation d'anticorps contre des antigènes exprimés sur la surface de lymphocytes B et non présents sur les lymphocytes T humains normaux, pour la préparation de compositions destinées à traiter la maladie greffe contre hôte chez les patients qui ont reçu des lymphocytes T transduits avec de tels antigènes.

2. Utilisation selon la revendication 1, dans laquelle des anticorps contre l'antigène CD20 de surface et des lymphocytes transduits avec l'antigène CD20 sont employés.

3. Utilisation selon la revendication 2, dans laquelle l'anticorps anti CD20 est un anticorps monoclonal humanisé.

4. Lymphocytes T humains transduits avec des antigènes exprimés sur la surface de lymphocytes B et non présents sur les lymphocytes T humains normaux.

5. Lymphocytes T selon la revendication 4 transduits avec un antigène CD20 humain.
